# EUROPEAN PATENT SPECIFICATION

(11) **EP 4 426 404 B1**
(45) Date of publication and mention of the grant of the patent: **08.04.2026**
(21) Application number: 22826472.7
(22) Date of filing: 03.11.2022
(51) Int. Cl.: A61M 21/00

(54) **SYSTEMS FOR COGNITIVE DISCONNECTION**
SYSTEME ZUR KOGNITIVEN TRENNUNG
SYSTÈMES DE DÉCONNEXION COGNITIVE

(30) Priority: 05.11.2021 US 202163276068 P
(43) Date of publication of application: 11.09.2024
(73) Proprietor: The Cleveland Clinic Foundation, Cleveland, OH 44195 (US); The United States Government as Represented by the Department of Veterans Affairs, Washington, DC 20420 (US)
(72) Inventor: MARASCO, Paul, D., Lakewood, OH 44107 (US); SCHOFIELD, Jonathon, S., Davis, CA 95618 (US); SHELL, Courtney, E., Lyndhurst, OH 44124 (US); THUMSER, Zachary, C., Cleveland, OH 44109 (US); BECKLER, Dylan, Lyndhurst, OH 44124 (US); SAKAI, Jonathan, Rocky River, OH 44116 (US)
(74) Representative: A.P.I. Conseil
(86) International application number: PCT/US2022/048824
(87) International publication number: WO 2023/081281

(56) References cited:
- KR-B1- 101 740 198
- HARA M ET AL: "A novel approach to the manipulation of body-parts ownership using a bilateral master-slave system", INTELLIGENT ROBOTS AND SYSTEMS (IROS), 2011 IEEE/RSJ INTERNATIONAL CONFERENCE ON, IEEE, 25 September 2011 (2011-09-25), pages 4664 - 4669, XP031959003, ISBN: 978-1-61284-454-1, DOI: 10.1109/IROS.2011.6048519

## Description

### BACKGROUND

Needle procedures (such as injections, IV placements, and venipunctures) are some of the most frequently performed clinical interventions, with blood draws occurring in nearly 40% of emergency department visits. Particularly for children, needle sticks induce pain in nearly 76% of pediatric patients and are described by children as one of the most traumatic aspects of visits to the hospital and doctor. Thus, these procedures can be painful and distressing, and lead to anxiety, loss of sleep, and resistance to medical visits. In view of this, effective needle pain management is an important driver of whether a patient will receive adequate healthcare as a child and throughout their adult life. Indeed, needle pain and the accompanying distress are well-documented contributors to healthcare avoidance behaviors and vaccine refusal in children and their guardians, as well as later in life when the child becomes an adult. The efficacy of conventional needle pain management strategies, which may include topical anesthesia, tactile stimulation, and conversational distraction techniques, remains debated and the challenges surrounding needle pain are still a pervasive clinical issue.

Separately, seizure disorders (e.g., epilepsy) chronically affect tens of millions of people. Individual seizures are caused by abnormally neuronal activity in the brain, and can be evidenced by decreased consciousness, automatism, tonic-clonic convulsions, and the like. Because of these outward symptoms, seizure disorders can affect an individual's ability to work and perform daily functions, and can be stigmatized.

Some solutions have been disclosed such as in the patent document KR101740198 which discloses a training system of brain computer interface or in Hara M et al. "A novel approach to the manipulation of body-parts ownership using a bilateral master-slave system" published during the International Conference on Intelligent robots and systems (IROS), 2011, IEE RSJ.

### BRIEF SUMMARY

According to a first aspect, the invention is about a system comprising:
a lower frame;
an upper frame rotatably attached to the lower frame;
a real plate attached to the lower frame and configured to hold a hand or a foot of a user;
a proxy plate attached to the upper frame; and
a proxy hand held on the proxy plate when the real plate is configured to hold the hand of the user or a proxy foot held on the proxy plate when the real plate is configured to hold the foot of the user,
wherein the real plate comprises a plurality of first sensory actuators and the proxy plate comprises a plurality of second sensory actuators, and
wherein each one of the second sensory actuators is at a location of the proxy hand or foot corresponding to a location of one of the first sensory actuators of the user's hand or foot; and
a processor configured to:
   simultaneously operate one of the first sensory actuators and the one of the second sensory actuators at the corresponding location of the proxy hand or foot,
   operate one of the first sensory actuators and the one of the second sensory actuators at the corresponding location of the proxy hand or foot at different times, or
   simultaneously operate one of the first sensory actuators at a location of the hand or foot of the user and one of the second sensory actuators at a different location of the proxy hand or foot,
   wherein an angle between the lower frame and the upper frame is greater than zero degrees such that when the sensory actuators are operated, the user's hand or foot and the first sensory actuators are not visible to the user, and the proxy hand or foot and the second sensory actuators are visible to the user.

According to the system of the invention, it may comprise the following optional features:
- Each of the first sensory actuators is at a different finger of the user's hand when the real plate is configured to hold the hand of the user or at a different toe of the user's foot when the real plate is configured to hold the user's foot, and each of the second sensory actuators is at a different finger or toe of the proxy hand or foot.
- The real plate and the proxy plate each comprise a plurality of finger clips attached to the respective hand or foot plate, each of the plurality of finger clips housing a different one of the first and second sensory actuators and being configured to hold a finger of the user's hand when the real plate is configured to hold the hand of the user or being configured to hold a toe of the user's foot when the real plate is configured to hold the user's foot, or the proxy hand or foot.
- Each of the first and second sensory actuators is a rack and pinion actuator comprising a servo motor configured to rotate the pinion.
- The proxy hand or foot is a digital representation of the hand or foot of the user on a display, the display being held on the proxy plate.

According to a second aspect, the invention is about a system comprising comprising:
a touch feedback device comprising a plurality of first sensory actuators and configured to hold an extremity of a user;
a proxy extremity; and
a processor configured to operate one of the first sensory actuators at a location of the extremity of the user corresponding to an identified location of the proxy extremity,
wherein the extremity of the user and the proxy extremity are in a visually separated fields of view, and
wherein the plurality of first sensory actuators are configured to induce a sensory response of the user when operated.

According to the system of the second aspect of the invention, it may comprise the following optional features:
- A wand,
   wherein the processor is configured to determine the identified location of the proxy extremity based on a proximity or touch of the wand to the proxy extremity.
- Predict the identified location of the proxy extremity based on a movement of the wand or the proximity of the wand to the proxy extremity; and operate the one of the first sensory actuators prior to the wand touching the proxy extremity.
- The proxy extremity is a digital representation of the extremity of the user.
- The touch feedback device is configured to hold a hand or a foot of the user.

### BRIEF DESCRIPTION OF THE SEVERAL VIEWS OF THE DRAWING

Figure 1 is a model of the relationship between a person's internally modeled reality, an externally applied reality (e.g., an illusory reality), and actual reality.
Figure 2 illustrates example disembodiment device according to the present disclosure.
Figure 3 illustrates a top view of the lower frame of the example disembodiment device of Fig. 2.
Figures 4A and 4B illustrate top and bottom views, respectively, of the upper frame of the example disembodiment device of Fig. 2.
Figures 5A and 5B illustrate hand plates for real and proxy hands, respectively, of the example disembodiment device of Fig. 2.
Figures 6A, 6B, and 6C illustrate front, side, and perspective views of an example finger clip of the hand plates of the example disembodiment device of Fig. 2.
Figure 7 illustrates an example application of the example disembodiment device of Fig. 2.
Figure 8 illustrates a folded carriable configuration of the example disembodiment device of Fig. 2.
Figure 9 illustrates a model of the relationship of individual modeled realities of two agents cooperating in a shared actual reality.
Figure 10 illustrates an additional example cognitive illusion system according to the present disclosure.

### DETAILED DESCRIPTION

The systems and methods (methods are not part of the invention as claimed) of the present disclosure induce a 'cognitive/perceptual illusion' that can be used to reduce pain during needle procedures and diagnose and treat seizure disorders, such as epilepsy. The systems and methods are also applicable to other outpatient procedures including, but not limited to, grafts, minor excisions, trauma/wound treatments, stitching, and the like, which can cause pain to the user.

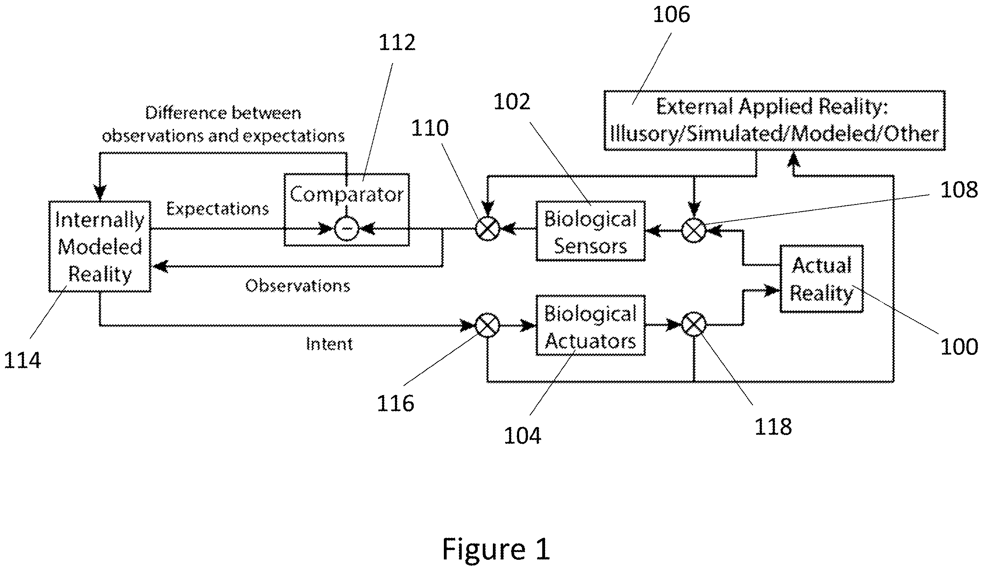

The cognitive illusion is based on the relationship between a person's internally modeled reality, an externally applied reality (e.g., an illusory reality), and the actual reality. This relationship is modeled in Fig. 1. Therein, the actual reality 100 relates to a state of actual reality that an individual samples by way of their biological sensors 102, and interacts with by way of their biological actuators 104. The biological sensors 102 may be, for example, the individual's sensory receptors such as for touch, taste, sight, smell, and sound; and the biological actuators 104 may be, for example, those related to motion control such as movement of the individual's arms, hands, legs, and feet (e.g., controlled by muscle action).

The reality sampled by biological sensors 102 may be modified by an externally applied reality 106, where valve 108 represents the combination of the actual reality 100 and the externally applied reality 106. The actual reality 100 combined with the externally applied reality 106 combined at valve 108 are together sampled by the biological sensors 102. Modifications of the sampled reality can include, but are not limited to, inputs such as tactile, visual, auditory, olfactory, thermal, nociceptive, proprioceptive, and gustatory either alone or in relation to anticipated outcomes.

The output of the biological sensors 102 can again be modified by the externally applied reality 106 (at valve 110) before being sent through the individual's nervous system to comparator 112. Modifications through valve 110 can include, but are not limited to, stimulatory approaches such as electrical, magnetic, chemical, mechanical, illusory, ultrasound, optical, thermal, anesthetic, and nerve redirection. In other words, these modifications are not those that would be sampled by biological sensors 102. Rather, the modifications introduced at valve 110 are to the outputs of the biological sensors 102 themselves (e.g., to the electrophysiological signals generated by the sensors 102).

The internally modeled reality 114 then is the individual's ideas, assumptions, predictions, and the like about the individual's place. This internally modeled reality 114 is created from the senses, observations, and learned experiences, and provides the individual with a framework by which to anticipate, predict, expect, and/or modulate their engagement with their reality.

The comparator 112 continuously monitors sensory information for discrepancies from internally modeled expectations 114. Resulting differences between observations (from biological sensors 102) and expectations (of the internally modeled reality 114) are used to update the internally modeled reality 114. In other words, the internally modeled reality 114 is composed of the differences between observations and expectations arising from the comparator 112 and generates and/or modifies expectations or intent.

The output of the individual's internally modeled reality 114 is supplied to the biological actuators 104, as a person intends to interact with the actual reality 100. The output of the internally modeled reality 114 may be combined with (and thus modify) the externally applied reality 106 at valve 116, prior to being supplied through the individual's nervous system to the biological actuators 104. Similar to internal modifications of the biological sensors at valve 110, modifications through valve 116 can include, but are not limited to, stimulatory approaches such as electrical, magnetic, chemical, mechanical, illusory, ultrasound, optical, thermal, anesthetic, and nerve redirection.

Similarly, the output of biological actuators 104 may modify the externally applied reality 106 at valve 118. Such modifications can alter the individual's movements, and change physical outcomes. These changes affect the actual reality 100, which is then detected by biological sensors 102 as described above.

As this relates to pain induced by medical procedures (e.g., needle procedures), the model of Fig. 1, which is not part of the invention as claimed, can be exploited by generating a cognitive illusion of ownership over a proxy extremity by providing discordant touch and visual information. It should be understood that while the present disclosure generally relates to procedures on a hand, it should be understood that the systems and methods of the present disclosure are applicable to any extremity or limb, such as fingers, hands, toes, feet, legs, arms, and the like. Further, it should be understood that the proxy extremity may be a prosthetic, digital representation (e.g., on a tablet or like display screen), or the like.

In generating the cognitive illusion of ownership over a proxy hand, a user feels a touch on their real hand, but sees the touch occurring on the proxy hand. This mismatch enters the model of Fig. 1 at valve 108, where the biological sensors 102 in the fingertips feel touch in one place (the user's real hand), and the biological sensors 102 of the eyes see touch occurring in a different place (the proxy hand). The comparator 112 then registers the mismatch between the seen touch and the felt touch, and the internally modeled reality 114 is updated to move the felt location of the real hand to the seen location of the proxy hand. This results in the user embodying the proxy hand, thereby disembodying (cognitively neglecting) the real hand. With the user's real hand disembodied, pain and other negative sensations caused by procedures performed on the real hand can be mitigated.

According to some embodiments which are not part of the invention as claimed, the user's hand is placed into a disembodiment device that holds both the user's hand and fingertips, and the hand and fingertips of a proxy hand. More particularly, the user's hand remains flat on a table with the palm up, while the proxy hand is also arranged palm up and oriented at an angle such that the proxy hand is visible to the user while obscuring the real hand. In this way, the proxy hand represents an externally applied reality 106 that replaces the user's real hand as detected by the biological sensors 102 (the user's eyes). In other words, as detected by the biological sensors 102, the actual reality 100 (the user's real hand) is modified by the externally applied reality (the proxy hand) at valve 108.

The disembodiment device has a first set of sensory actuators associated with the real hand, and a second set of sensory actuators associated with the proxy hand. The first set of sensory actuators is operated to cause a tactile sensory response on the user's real hand, but cannot be seen by the user. Conversely, the second set of sensory actuators is operated to be seen by the user to be engaging with the proxy hand, but does not cause a tactile sensory response. In other words, operation of the sensory actuators results in activation of the biological sensors 102 in the real hand (e.g., to produce a haptic feeling). In this regard, the sensory actuators may take any form that would cause a sensory response. For example, each sensory actuator may be a device that directly touches the real hand, or one that results in the sensation of a touch (e.g., by releasing pressurized air, or increasing air pressure at the hand, or the like).

Again operating at valve 108 to modify the actual reality 100, the first set of sensory actuators is synchronously (at the same time and relative place on the corresponding hand) operated with the second set of sensory actuators in the case of reducing pain during a medical procedure (e.g., needle procedures). The timing and location of sensory actuator operation may be random and last on the order of a few seconds to a few minutes. This induces a sensory mismatch between the visual and tactile sensory information streams from biological sensors 102 that are read and compared at the user's brain (the comparator 112). The sensory mismatch between what is seen and what is felt induces conflict in the user's internally modeled reality 114.

To resolve the conflict between what is seen and what is felt, the expectations generated by the internally modeled reality 114 are realigned with respect to the observed sensory mismatch to minimize the discrepancy identified by comparator 112. This cognitive realignment of the user's internally modeled reality 114 shifts attribution of the sensation of the felt touch on the user's real hand to the location of the proxy hand that is in view. The realignment of felt touch to the proxy hand causes the cognitive illusion that the proxy hand is part of the user's body.

Simultaneously, the realignment of felt touch to the proxy hand leads to cognitive neglect of the user's real hand and disembodiment of the user's real hand from their body image. As a result, pain felt by a needle (or other procedure) in the user's real hand is reduced.

And as this relates to the diagnosis and treatment of seizure disorders such as epilepsy, a comparator 112 of an epileptic brain is less stringent when resolving conflict between what is seen and what is felt. In this case the requirement of specific context related to the simultaneous presentation of both seen and felt sensations for cognitive realignment is lost. As such, simple correlations in time (such as seen touches on the proxy hand that follow felt touches to the real hand by a brief yet equidistant interval) or space (such as seen touches on a digit of the proxy hand that are simultaneously felt on a different digit of the real hand) results in maladaptive cognitive realignment of the user's internally modelled reality 114, which shifts attribution of the sensation of the felt touch on the user's real hand to the location of the proxy hand in view.

In the case of diagnosing and/or treating epilepsy, the first set of actuators is asynchronously operated with the second set of actuators. Particularly, operation of the first and second sets of actuators is offset either temporally or spatially. The shift to attribution of the sensation of the felt touch on the user's real hand to the location of the proxy hand in view under temporal or spatial mismatch thus reveals the maladaptive propensity for updating the user's internally modelled reality 114 based on non-contextual simple visual and temporal correlations.

Considering this, when diagnosing and/or treating epilepsy, the first and second sets of sensory actuators would not operate synchronously as described above. Rather, the sensory actuators are operated asynchronously, where operation of a pair of sensory actuators (e.g., one of the first set of sensory actuators and one of the second set of sensory actuators) are separated in time or space. For example, a pair of sensory actuators associated with the same digit are operated at different times. Alternatively, a pair of sensory actuators associated with different digits are operated at the same time. Because spatial distance can affect the comparator 112 of the epileptic brain, the digits associated with a pair of sensory actuators operated at the same time may be selected based on their relative locations.

In one example embodiment, which is not part of the invention as claimed, digits D1-D3 (thumb, index, and middle, respectively) may be characterized as a first group and digits D4-D5 (ring and little/pinky, respectively) may be characterized as a second group. In such an embodiment, the sensory actuator of each pair may be associated with a digit in different groups. For example, the sensory actuator from the first set of sensory actuators may be associated with digit D2 and the sensory actuator from the second set of sensory actuators may be associated with digit D4. In this case, the sensory actuator associated with user's real digit D2 is operated at the same time the sensory actuator associated with the proxy digit D4 is operated.

According to another example embodiment, which is not part of the invention as claimed, a first digit is selected (e.g., by random) and a second digit is then determined as the digit that is farthest away. For example, if digit D1 is selected on the user's real hand, then digit D5 is selected on the proxy hand. Thus, the sensory actuator associated with digit D1 of the first set of sensory actuators is operated at the same time the sensory actuator associated with digit D5 of the second set of sensory actuators is operated. In still another embodiment, there is at least one digit between the digits associated with the sensory actuator pair. In other words, sensory actuators are not operated at the same time for the same or adjacent digits. For example, the sensory actuator pairs may be associated with digits D1 and D3, D1 and D4, D1 and D5, D2 and D4, D2 and D5, or D3 and D5.

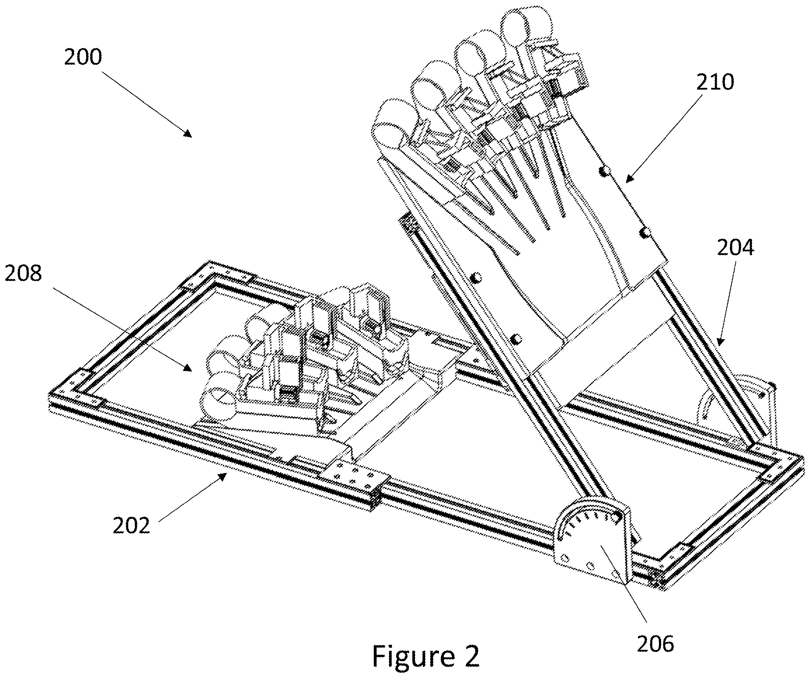

An example disembodiment device 200 according to the invention is illustrated in Fig. 2. The device 200 has a lower frame 202 and an upper frame 204 rotatably mounted to each other at one end by a hinge 206. As seen in the figure, the upper frame 204 may be rotatable to about 90 degrees with respect to the lower frame 202. Rotation of the upper frame 204 relative to the lower frame 202 may be manual, or automated/electrically controlled. A hand plate 208 that supports and holds the user's real hand is attached to the lower frame 202; and similarly, a hand plate 210 that supports and holds the proxy hand is attached to a top side of upper frame 204. In embodiments where the proxy hand is digitally represented, the upper frame 204 may hold a tablet or like display screen that displays the digital representation of the proxy hand. An electronics enclosure (not visible) for operating the disembodiment device 200 is attached to a bottom side of upper frame 204.

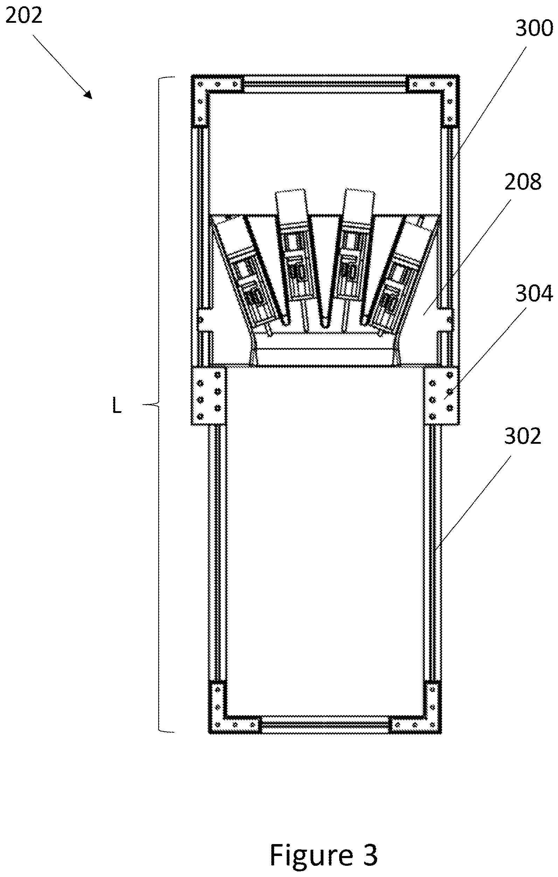

Figure 3 illustrates a top view of the lower frame 202. As seen therein, the lower frame 202 includes an upper frame portion 300 and a lower frame portion 302. Each frame portion 300, 302 is substantially square or rectangular with three sides (thus having an open end). The open ends of the frame portions 300, 302 are connected to each other by brackets 304. The hand plate 208 for the real hand is connected to the upper frame portion 300.

The frame portions 300, 302 preferably have tracks (e.g., extrusions, cutouts, grooves, rails, or the like) to which the hand plate 208 and brackets 304 can be attached. Accordingly, the attachment points of the hand plate 208 and brackets 304 to each portion 300, 302 can be adjusted along to any point of the frame portions 300, 302 to suitably accommodate a user's hand and arm. In other words, a total length L of the lower frame 202, and relative position of hand plate 208, can be adjusted according to a user's arm and hand length/size.

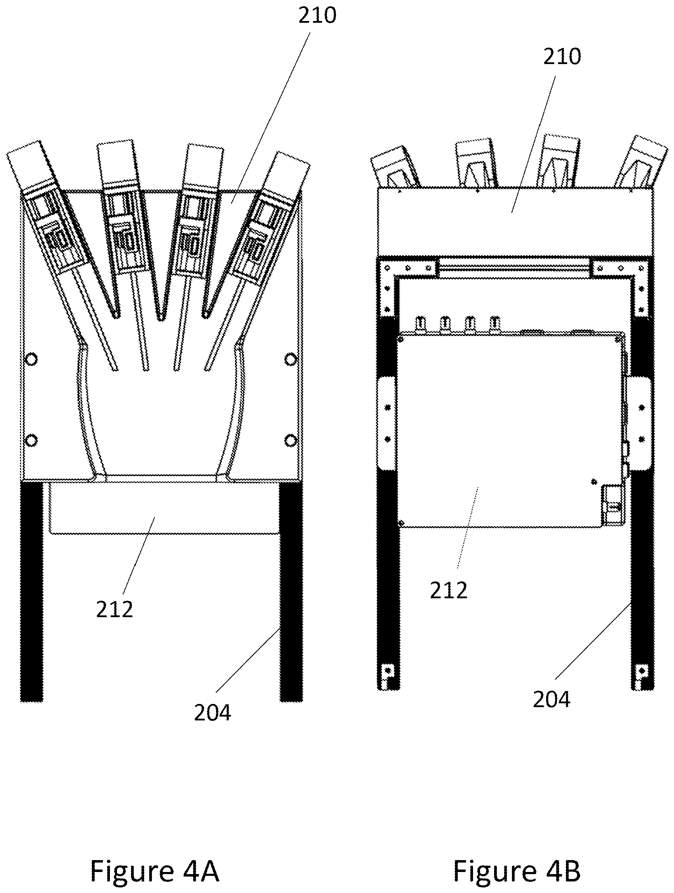

Figures 4A and 4B illustrate top and bottom views, respectively, of the upper frame 204. Similar to the lower frame 202, hand plate 210 is attached to a top side of the upper frame 204. The electronics enclosure 212 is attached to a bottom side of the upper frame 204. As with the connections to lower frame 202, the upper frame may also include tracks to which the hand plate 210 and electronics enclosure 212 may be attached, so that their locations are adjustable.

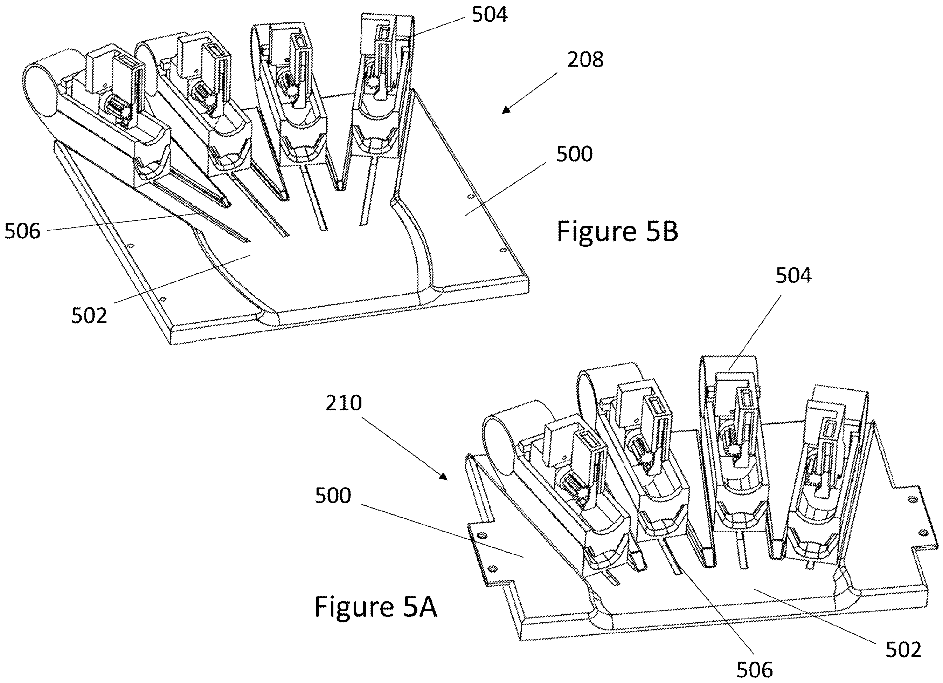

Figures 5A and 5B further illustrate hand plates 210 and 208, respectively, in more detail. Each hand plate includes a base 500, which is attached to the corresponding frame 202, 204. The base 500 may include a cutout 502, extrusion, or the like that substantially corresponds to the shape of the hand. The cutout 502 helps properly align and secure the user's real hand (or the proxy hand) snuggly with the base 500.

The hand plates 208, 210 further include finger clips 504 attached to the plates 208, 210 in a portion of the cutout 502 corresponding to at least one finger. Although the example of Figs. 5A and 5B illustrate four finger clips 504 (one associated with each finger other than thumb), any number may be utilized. The finger clips 504 further secure the user's real hand or the proxy hand to the corresponding base 500, and house the sensory actuators that touch the hand. The finger clips 504 are attached to the base 500 in tracks 506. In this manner, the length of the finger can be adjusted to fit a user's hand size by moving finger clips 504 along the track.

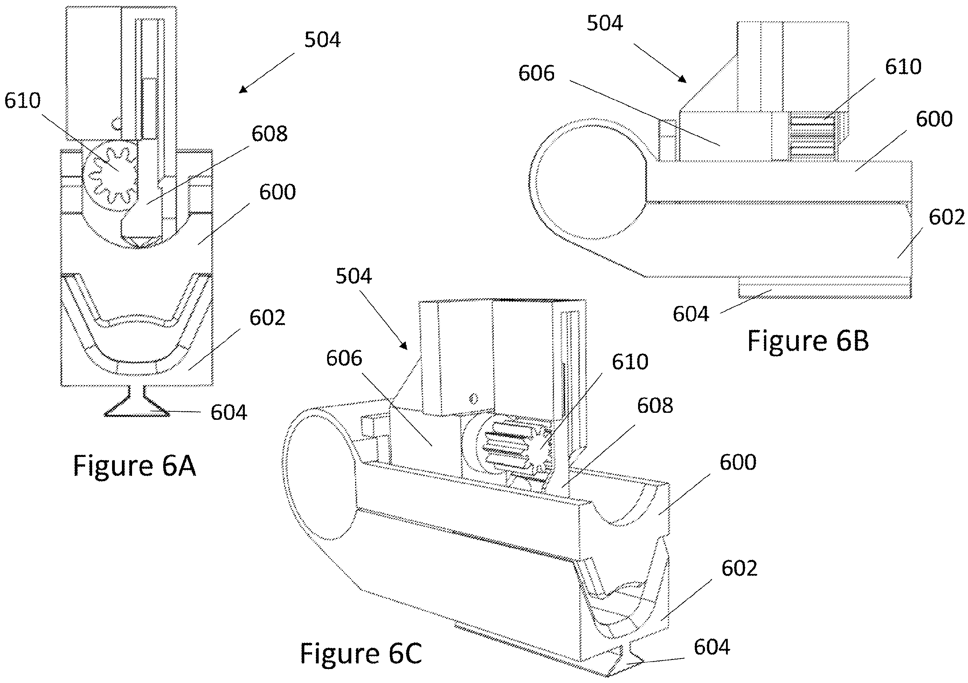

The finger clips 504 are illustrated in more detail in Figs. 6A-6C, which show front, side, and perspective views, respectively. The finger clips 504 include an upper portion 600 and lower portion 602, which are configured to hold a finger therebetween. For example, the upper portion 600 and lower portion 602 may be spring-loaded or otherwise biased in a closed state so as to pinch the finger from the top and bottom. A dove-tail 604 or like element underneath the lower portion 602 can engage with the tracks 506 of the plates 208, 210. In other words, the finger clips 504 may be attached to the plates 208, 210 with a sliding dovetail joint.

The upper portion 600 of the finger clip 504 may include a slot or like aperture through which the sensory actuator may engage with the finger. In the example of Figs. 6A-6C, the sensory actuator is of a plunger-type and includes a servo motor 606, which drives a rack 608 and pinion 610. The servo motor 606 drives rotation of the pinion 610, which causes the rack 608 to move up and down (plunge) through the aperture in the upper portion 600 of the finger clip 504. While a rack and pinion actuator is described herein, it should be understood that any actuator may be utilized. For example, the sensory actuator may be of a piston-type actuator in which hydraulic, pneumatic, or an electrical motor cause a piston to extend from a cylinder towards the finger. In some examples, one or more of the sensory actuators may be mechanically linked for example by a cam shaft causing synchronized motion of the pistons. In still other examples, the sensory actuator may release pressurized air (e.g., by opening a valve), or otherwise cause a burst of pressurized air, toward the finger. In these examples, the sensory actuator itself does not directly engage the finger. Preferably the sensory actuator is visible to the user so that they may see when and where actuation occurs.

Operation of the disembodiment device 200 may be wholly or partially controlled by electronics in the electronics enclosure 212. The electronics enclosure 212 may house at least a processor, memory, and power source. The power source may be an internally housed battery or a power supply connectable to a mains power (e.g., a wall outlet). The processor is configured to output control signals to each of the sensory actuators of the device 200. These control signals may be supplied to the sensory actuators, for example, via communication cables removably attached to the electronics enclosure 212 at one end, and to the sensory actuators of the corresponding hand plates 208, 210 at another end, via communication ports at the electronics enclosure 212 and the individual sensory actuators or the plates 208, 210.

The processor may further be configured to communicate with external or otherwise remote devices through wired or wireless connections. For example, the processor may be further configured to control operation of lights associated with each sensory actuator. Control signals for the lights may then be supplied from the electronics enclosure 212 via expansion ports of the electronics enclosure. In other examples, the processor may be configured to communicate with a remote control processor (e.g., a remote server) via a wired connection or wireless network. In this manner, the disembodiment device 200 may be remotely operated or monitored.

The electronics enclosure 212 may further include human-machine-interface (HMI) elements. For example, power switches may control the supply of power to the processor (and thus the whole device) and/or the hand plates 208, 210 individually. Further, the position of each sensory actuator may be adjusted by knobs or the like, for example, by adjusting potentiometers. The sensory actuator position adjustment may allow for accommodation of different finger diameters and placements within the finger clips 504. Furthermore, the sensory actuator position adjustment may allow for close proximity placement of the sensory actuator to the finger. The processor may be configured to adjust control of each sensory actuator (e.g., a power, speed, magnitude, or the like). Still further, a mode selection switch(es) may be used to select an operation program executed by the processor. Such programs may control the sequence, order, timing, and like operation of each of the sensory actuators. Changing the sequence, order, and timing of actuation may allow for modifications to the experience of the ownership illusion, and adjusting experimental controls and for exploring comparator function during research. For example, such modes can include a needle stick (or like) procedure mode in which the sensory actuators of each plate 208, 210 are operated synchronously, an epilepsy diagnostic or treatment mode in which the sensory actuators of each plate 208, 210 are operated asynchronously, and/or a research mode in which the sensory actuators of each plate 208, 210 can be specially controlled according to a research protocol.

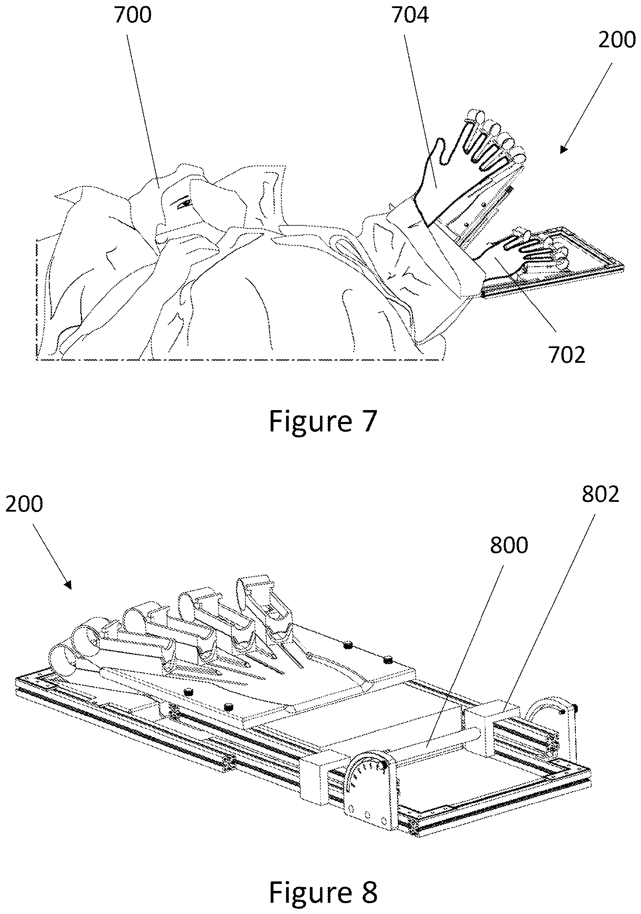

With reference to Fig. 7, the disembodiment device 200 may be employed for example in a surgical setting. In such an environment, a user 700 is on their back, and their real hand 702 is held to the hand plate 208 of the lower frame 202 of the disembodiment device 200, with their four fingers (which may include or exclude the thumb) held in the corresponding finger clips 504. A proxy hand 704 (and the fingers thereof) is similarly held in plate 210 of the upper frame 204. Preferably the skin tone of the proxy hand 704 substantially matches that of the user's real hand 702 to further strengthen the visual illusion of the proxy hand. The upper frame 204 is raised at approximately a 60 degree angle with respect to the lower frame 202.

In the configuration of Fig. 7, only the proxy hand 704 is visible to the user 700. The processor of the disembodiment device 200 may control operation of each sensory actuator according to a program, for example, so that sensory actuators of corresponding fingers of the real hand 702 and proxy hand 704 are synchronously operated. As discussed above, this induces the cognitive illusion that causes the user to embody the proxy hand 704 and disembody the real hand 702.

When not in use, the disembodiment device 200 may be folded and carriable. As illustrated in Fig. 8, the upper frame 204 is rotated such that the angle between the upper frame 204 and lower frame 202 approaches zero degrees. In other words, the upper frame 204 may be folded down so that it essentially rests on top of the lower frame 202. A carry bar 800 spanning a width of the disembodiment device may be attached to the upper and lower frames 202, 204 by clips 802. In addition to attaching the carry bar 800 to the device 200, the clips 802 further serve to constrain rotation of the upper frame 204 and prevent the device 200 from opening. The carry bar 800 may be used as a handle to carry or otherwise transport the device 200.

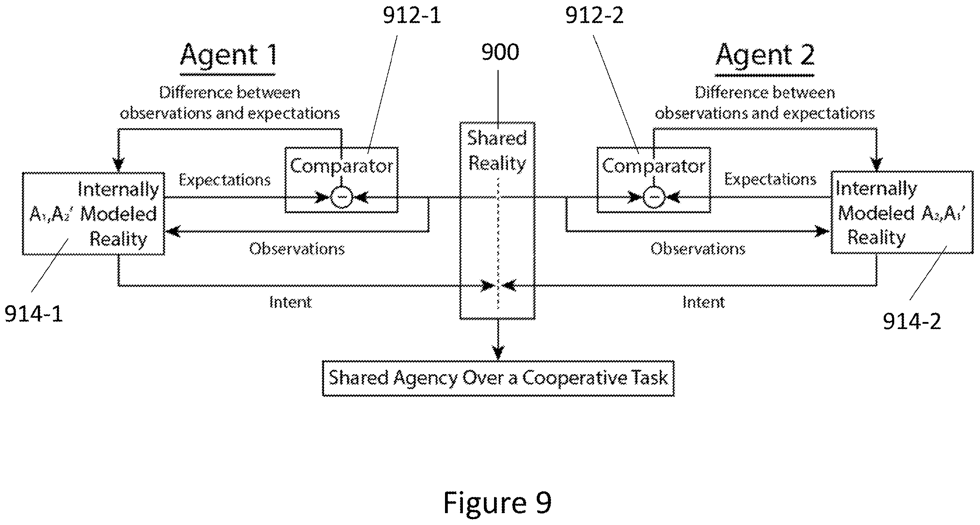

Turning now to Fig. 9, the model of Fig. 1 may be reimagined for two cooperative agents. As used herein, an agent may be a human, intelligent machine, HMI, or the like that can sense and/or interact with an actual reality. In essence, Fig. 9 represents two agents, each modeled according to Fig. 1, but sharing a common actual reality. More particularly, as seen in Fig. 9, each agent (Agent 1, Agent 2) shares a common actual reality 900. This shared reality 900 is analogous to the actual reality 100 of Fig. 1, which is detectable by each agent's sensors (e.g., biological sensors 102 in the case of a human agent, or transducer such as a touch sensor, light sensor, camera, microphone, or the like in the case of an intelligent machine or HMI).

The sensed shared reality of each agent is then compared at comparator 912-1, 912-2 (analogous to comparator 112 of Fig. 1) with the agent's expectations based on their internally modeled reality 914-1, 914-2 (analogous to the internally modeled reality 114 of Fig. 1). Each agent may then interact with and affect the shared reality 900 with their actuators (e.g., biological actuators 104 in the case of a human agent, or electrical or mechanical actuators such as displays, motors, speakers, or the like in the case of an intelligent machine or HMI) based on their internally modeled reality 914-1, 914-2.

Because both agents interact with the same shared reality 900, the internally modeled reality for each agent is affected by the internally modeled reality of the other agent. For example, the internally modeled reality 914-1 of Agent 1 corresponds to shared reality 900 as modified by Agent 2 (based on the internally modeled reality 914-2 of Agent 2) and sensed by Agent 1. In this sense, action of each agent on the shared reality has a similar effect to the externally applied reality 106 of Fig. 1.

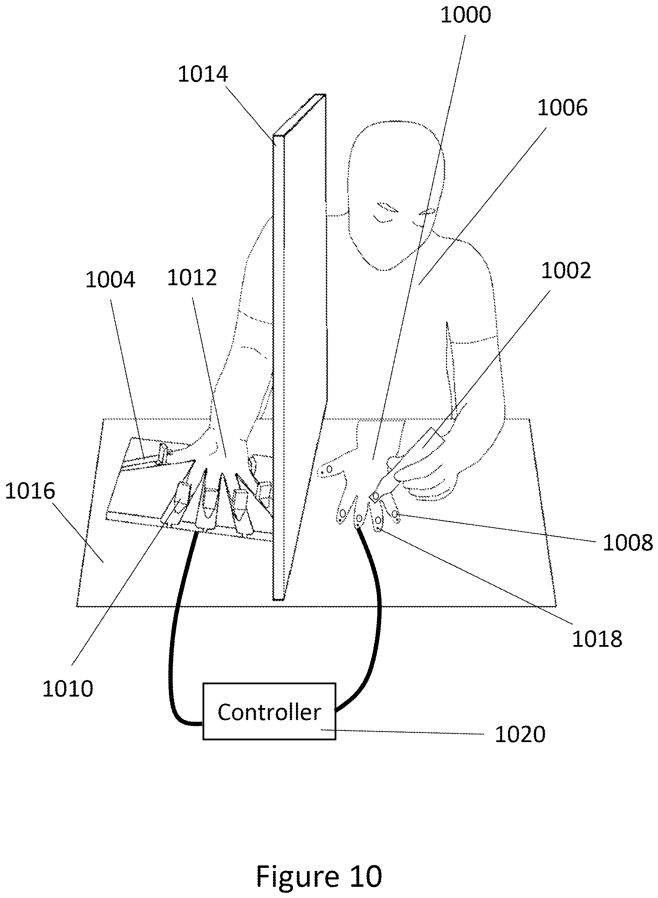

Based on the model of Fig. 9, other embodiments of the present disclosure may realize the cognitive illusion described herein according to the example of Fig. 10. More particularly, the example of Fig. 10 utilizes a sensorized proxy (e.g., rubber) limb 1000, a touch device (e.g., a wand) 1002, a controller (e.g., a processor, computer, discrete controller, or the like) 1020, and a touch feedback device 1004 (collectively corresponding to Agent 2 of Fig. 9). The sensorized proxy limb 1000 may also be embodied in a digital/electronic form such as the display of an animated artificial hand on a screen such as a tablet device or smartphone.

In operation, a user 1006 (corresponding to Agent 1 of Fig. 9) uses the wand 1002 to touch the proxy limb 1000. The proxy limb 1000 and/or the wand 1002 may include sensors 1008 such as touch, pressure, capacitive, proximity sensors (e.g., infrared), accelerometers, and/or the like in the tip of the wand 1002 and at the fingertips or other locations of the proxy limb 1000. For example, in some embodiments, the proxy limb 1000 may have proximity or touch sensors to detect an actual touch or proximity of the wand 1002 on the proxy to preemptively infer an anticipated touch. Alternatively or additionally, the wand 1002 may include a pressure sensor to detect the strength of the touch and/or accelerometers to determine movement characteristics of the wand 1002 as it is controlled by the user to preemptively infer the anticipated touch. In short, the sensors 1008 are configured to detect the touch and/or presence of a wand 1002 controlled by the user 1006 with a non-procedure hand.

In some embodiments such as that in Fig. 10, the touch feedback device 1004 may be similar to the hand plate 208 for user's real hand discussed above. For example, the touch feedback device 1004 may include sensory actuators 1010 (e.g., vibration devices, servo motors, piezoelectric elements, and the like) at locations corresponding to the sensors in the proxy hand (e.g., at the finger tips). Each sensor 1008 of the proxy hand 1000 is then coupled to its corresponding actuator 1010 in the touch feedback device 1004 via the controller 1020.

For example, if the proxy limb 1000 includes sensors 1008 at each fingertip, the touch feedback device 1004 preferably has actuators 1010 at locations in contact with the user's fingertips. In some embodiments, the touch feedback device 1004 may include finger clips or like devices for holding the user's real hand in the touch feedback device. Those finger clips may house the sensory actuators 1010, similar to the finger clips 504 discussed above. In other embodiments, the touch feedback device 1004 may be embodied as a glove.

Accordingly, upon touching the proxy limb 1000 with the wand 1002, the touch feedback device 1004 synchronously touches the user's real limb 1012 at the corresponding location. For example, if the user 1006 touches the wand 1002 at an index fingertip on the proxy limb 1000, the touch feedback device 1004 preferably actuates a sensory actuator 1010 in contact with the user's real index finger. The cooperation between the detection of the wand 1002 by the proxy limb 1000 and actuation of the sensory actuators 1010 in the touch feedback device is moderated by the controller. While the controller 1020 is shown in Fig. 10 as a separate control device connected between the proxy hand 1000 and the touch feedback device 1004 (in a wired or wireless manner), it should be understood that the controller may be part of the proxy hand 1000 or the feedback device 1004, with the proxy hand 1000 and feedback device 1004 directly connected to each other in a wired or wireless manner.

The user's use of the wand 1002 may be part of an interactive game. In one embodiment, the user 1006 places their real hand (on the limb the procedure is to be performed) 1012, palm up, into the touch feedback device 1004, or in a glove of the touch feedback device 1004. The touch feedback device 1004 may be hidden behind a screen 1014 affixed to a procedure table 1016, or otherwise in a visually separate field of view than the proxy hand 1000. The sensory actuators 1010 are located at each fingertip position of the feedback device 1004. The proxy or like proxy hand/arm 1000 is provided on a side of the screen 1014 that the user can see, and is posed in the same way that the hidden real hand 1012 is posed in the feedback device 1004.

In use, as noted above, when the user 1006 touches the wand 1002 to one of the fingertips of the proxy hand 1000, the wand 1002 is detected by the sensor 1008 of the proxy fingertip. In some embodiments discussed in more detail below, the wand 1002 only needs to be within a predetermined proximity of the fingertip to be sensed by the sensors 1008. The sensor 1008 then transmits a signal to the controller 1020, which identifies the location of the detection and causes actuation of the corresponding sensory actuator 1010 of the feedback device 1004. Depending on the sensors used, a pressure, proximity, or the like may also be detected and analyzed by the controller 1020 so as to cause a haptic sensation caused by the sensory actuator 1010 to more closely correspond to the actual touch by the wand 1002. The wand, in turn, may contain actuators that use the predictive nature of the proximity sensing and touch feedback to prepare the system to provide low (or negligible) latency to the feedback device 1004 that give the user 1006 the impression of fingertip compliance. Such a realistic latency can provide a more realistic perceptual illusion of game and hand naturalness to facilitate a cooperative engagement with the cognitive mechanism (the shared reality 900).

For example, the controller 1020 may cause a high intensity actuation upon a pressure sensor's detection of a high pressure touch by the wand 1002. Preferably, the controller 1020 causes actuation of the sensory actuators 1010 simultaneously (or nearly simultaneously, if realistic latency based on predictive touch is employed) with the touch of the wand 1002 to the proxy 1000. As discussed above, such simultaneous touch and feedback further strengthens the illusion and the ability of the user to cognitively neglect the real procedure hand. With respect to Fig. 9, this effect can be understood as the patent's observations aligning with expectations through the comparator 912-1 to modulate internally modeled reality 914-1 and update the shared reality 900 through the user's intent, whereas the Agent 2 devices of Fig. 10 provide information in cooperation with the user 1006 to facilitate the perceptual illusion of hand naturalness. Thus, both the user 1006 as Agent 1 and the devices of Fig. 10 as Agent 2 share agency over the cooperative task of perception of naturalness of interaction between the user and devices.

In some embodiments, the proxy hand 1000 may further include LEDs or like lights 1018 to indicate particular locations of the proxy hand 1000 for the user 1006 to touch with the wand 1002. For example, lights 1018 in each finger may be illuminated (as controlled by the controller 1020) according to a pattern in which the user is to touch each finger of the proxy hand 1000. The pattern may show only one light at a time-for example, thus waiting for a user 1006 to touch the finger corresponding to the illuminated light before illuminating another light-or may show a plurality of lights at a time-for example, requiring the user 1006 to remember a series of touches to perform after the illumination pattern is shown. Characteristics of the lights (e.g., color and intensity) may also be controlled to indicate a desired pressure of touch, speed of touch, or the like, or to help the user 1006 distinguish between the different fingers. The lights 1018 may be illuminated randomly or according to a predetermined pattern.

By performing dissociation with the above or a like game causes the user 1006 to further focus cognitive attention on the dissociation process. Further, this limits the interruption of clinical workflow because the user is in charge of establishing the cognitive disconnection while waiting for a clinician to prepare a workspace. Still further, presenting the dissociation as a game can be fun and distract the user from the impending procedure.

Referring back to Figs. 1 and 9, the brain is highly sensitive to timing differences in the comparison of the internally modeled expected reality 114, 914-1 and the sensed reality output of valve 110 at comparator 112, 912-1. In view of this, touch events of the wand 1002 may be anticipated and compensated for by speeding up the actual sensation through feedback device 1004. In other words, unnatural delays between when the user expects to feel a touch (based on their visual perception) of the wand 1002 touching the proxy 1000, and when they actually feel touch from the sensory actuator 1010 of the feedback device 1004 can affect the illusion. Merely making the system as fast as possible to disguise inherent lag does not necessarily produce a natural response, however. Further, using another human to administer the touches on both hands is not necessarily desirable due to delays in that human's perception and the required resources of relying on other personnel.

In view of this, the controller 1020 may control the sensory actuators based on projected anticipated touch events into the future in order to compensate for electrical, mechanical, and processing lag in the illusion game. For example, because proximity sensors can recognize the presence of the wand 1002 in the proximity of the fingertip of the proxy 1000 prior to an actual touch, the sensor 1008 may signal the controller 1020 of the impending touch. The controller 1020 can then properly time its output signal actuating the appropriate sensory actuator 1010 so that the actuation begins at the time of actual touch.

By knowing the location of each sensor 1008, the controller 1020 may identify the location of the wand 1002 based on which sensor 1008 senses the presence of the wand 1002. In some embodiments, the wand 1002 may be detected by multiple sensors 1008. In these cases, the controller 1020 may predict the location of the touch by identifying the sensor 1008 with which the proximity changes at the greatest rate, thus suggesting the sensor 1008 to which the wand 1002 is moving most directly toward.

In some embodiments, velocity, acceleration, and/or motion information of the wand 1002 may also be used by the controller 1020 to predict its location by recognizing that the user 906 may naturally (at least temporarily) slow movement of the wand 1002 as the wand 1002 approaches the desired touch location, in order to increase accuracy of the touch. Thus, a sensor 1008 detecting proximity at a time corresponding to a slowing of the wand 1002 may be predictive of the location of the future touch.

In some embodiments, the controller 1020 may predict the touch location based on activation of the above-discussed LEDs 1018. In other words, because the activated lights 1018 serve as instructions for body part locations to be touched by the user 1006, the controller 1020 may assume the user will follow the instructions and thus predict the location of a future touch based on the light instructions.

In still other embodiments, the controller 1020 may analyze sensor signals from the proxy limb 1000 and/or the wand 1002 to determine both a velocity/acceleration and directionality of the wand's movement. The controller 1020 may use the directionality information to predict the touch location, and the velocity/acceleration to predict the touch time. In short, the controller may predict when, and on which finger, an actual touch on the proxy hand 1000 will occur. The controller 1020 may then cause the sensory actuator 1010 for the corresponding finger on the real hand 1012 to actuate at the predicted time of touch.

In some embodiments, a system that compensates for lag is not limited to the full system lag or no lag at all. Rather, the lag may merely be reduced, zero, negative, dithered (e.g., having a zero-mean but deliberately noisy), and the like.

The controller 1020 may also be configured to learn the behavior of the user 1006, and adjust in real-time to make more accurate and effective predictions about the timing of future touch events. For example, by considering data from accelerometers or like sensors in the wand (e.g., as recorded in a memory), the controller 1020 may determine average rate of movement of the wand. By knowing the detection proximity of sensors 1008 in the proxy hand 1000, and the speed of the wand 1002, the controller may more accurately determine when the wand will touch the proxy. In some embodiments, the controller 1020 may be or include a learned controller including, for example, a machine learning system that is continually trained by the user's wand movement. Accordingly, the controller's predictions may improve during the illusion game. These predictions by the controller 1020 may also be unique to each user.

The present invention is defined by the appended claims.

## Claims

1. A system (200) comprising:
a lower frame (202);
an upper frame (204) rotatably attached to the lower frame (202);
a real plate (208) attached to the lower frame (202) and configured to hold a hand or a foot of a user;
a proxy plate (210) attached to the upper frame (204); and
a proxy hand held on the proxy plate (210) when the real plate (208) is configured to hold the hand of the user or a proxy foot held on the proxy plate (210) when the real plate (208) is configured to hold the foot of the user,
wherein the real plate (208) comprises a plurality of first sensory actuators and the proxy plate (210) comprises a plurality of second sensory actuators, and
wherein each one of the second sensory actuators is at a location of the proxy hand or foot corresponding to a location of one of the first sensory actuators of the user's hand or foot; and
a processor configured to:
simultaneously operate one of the first sensory actuators and the one of the second sensory actuators at the corresponding location of the proxy hand or foot,
operate one of the first sensory actuators and the one of the second sensory actuators at the corresponding location of the proxy hand or foot at different times, or simultaneously operate one of the first sensory actuators at a location of the hand or foot of the user and one of the second sensory actuators at a different location of the proxy hand or foot,
wherein an angle between the lower frame (202) and the upper frame (204) is greater than zero degrees such that when the sensory actuators are operated, the user's hand or foot and the first sensory actuators are not visible to the user, and the proxy hand or foot and the second sensory actuators are visible to the user.

2. The system (200) of claim 1, wherein each of the first sensory actuators is at a different finger of the user's hand when the real plate (208) is configured to hold the hand of the user or at a different toe of the user's foot when the real plate (208) is configured to hold the user's foot, and each of the second sensory actuators is at a different finger or toe of the proxy hand or foot.

3. The system (200) of claim 2, wherein the real plate (208) and the proxy plate (210) each comprise a plurality of finger clips (504) attached to the respective hand or foot plate (208, 210), each of the plurality of finger clips (504) housing a different one of the first and second sensory actuators and being configured to hold a finger of the user's hand when the real plate (208) is configured to hold the hand of the user or being configured to hold a toe of the user's foot when the real plate (208) is configured to hold the user's foot, or the proxy hand or foot.

4. The system of claim 2, wherein each of the first and second sensory actuators is a rack (608) and pinion (610) actuator comprising a servo motor (606) configured to rotate the pinion (610).

5. The system of claim 1, wherein the proxy hand or foot is a digital representation of the hand or foot of the user on a display, the display being held on the proxy plate (210).

6. A system (200) comprising:
a touch feedback device (1004) comprising a plurality of first sensory actuators and configured to hold an extremity of a user;
a proxy extremity; and
a processor configured to operate one of the first sensory actuators at a location of the extremity of the user corresponding to an identified location of the proxy extremity,
wherein the extremity of the user and the proxy extremity are in a visually separated fields of view, and
wherein the plurality of first sensory actuators are configured to induce a sensory response of the user when operated.

7. The system (200) of claim 6, further comprising:
a wand (1002),
wherein the processor is configured to determine the identified location of the proxy extremity based on a proximity or touch of the wand (1002) to the proxy extremity.

8. The system (200) of claim 6, wherein the processor is further configured to:
predict the identified location of the proxy extremity based on a movement of the wand (1002) or the proximity of the wand (1002) to the proxy extremity; and
operate the one of the first sensory actuators prior to the wand (1002) touching the proxy extremity.

9. The system (200) of claim 6, wherein the proxy extremity is a digital representation of the extremity of the user.

10. The system of claim 6, wherein the touch feedback device (1004) is configured to hold a hand or a foot of the user.

## Patentansprüche

1. System (200), das Folgendes umfasst:
einen unteren Rahmen (202);
einen oberen Rahmen (204), der drehbar am unteren Rahmen (202) befestigt ist;
eine reale Platte (208), die am unteren Rahmen (202) befestigt und dazu ausgelegt ist, eine Hand oder einen Fuß eines Benutzers zu halten;
eine Proxy-Platte (210), die am oberen Rahmen (204) befestigt ist; und
eine Proxy-Hand, die auf der Proxy-Platte (210) gehalten wird, wenn die reale Platte (208) dazu ausgelegt ist, die Hand des Benutzers zu halten, oder einen Proxy-Fuß, der auf der Proxy-Platte (210) gehalten wird, wenn die reale Platte (208) dazu ausgelegt ist, den Fuß des Benutzers zu halten,
wobei die reale Platte (208) eine Vielzahl von ersten Erfassungsaktuatoren umfasst und die Proxy-Platte (210) eine Vielzahl von zweiten Erfassungsaktuatoren umfasst, und
wobei sich jeder der zweiten Erfassungsaktuatoren an einer Stelle der Proxy-Hand oder des Proxy-Fußes befindet, die einer Stelle von einem der ersten Erfassungsaktuatoren der Hand oder des Fußes des Benutzers entspricht; und
einen Prozessor, der zu Folgendem ausgelegt ist:
gleichzeitiges Betätigen von einem der ersten Erfassungsaktuatoren und des einen der zweiten Erfassungsaktuatoren an der entsprechenden Stelle der Proxy-Hand oder des Proxy-Fußes,
Betätigen von einem der ersten Erfassungsaktuatoren und des einen der zweiten Erfassungsaktuatoren an der entsprechenden Stelle der Proxy-Hand oder des Proxy-Fußes zu verschiedenen Zeiten oder gleichzeitiges Betätigen von einem der ersten Erfassungsaktuatoren an einer Stelle der Hand oder des Fußes des Benutzers und einem der zweiten Erfassungsaktuatoren an einer anderen Stelle der Proxy-Hand oder des Proxy-Fußes,
wobei ein Winkel zwischen dem unteren Rahmen (202) und dem oberen Rahmen (204) größer ist als null Grad, derart, dass, wenn die Erfassungsaktuatoren betätigt werden, die Hand oder der Fuß des Benutzers und die ersten Erfassungsaktuatoren für den Benutzer nicht sichtbar sind und die Proxy-Hand oder der Proxy-Fuß und die zweiten Erfassungsaktuatoren für den Benutzer sichtbar sind,

2. System (200) nach Anspruch 1, wobei sich jeder der ersten Erfassungsaktuatoren an einem anderen Finger der Hand des Benutzers befindet, wenn die reale Platte (208) dazu ausgelegt ist, die Hand des Benutzers zu halten, oder an einem anderen Zeh des Fußes des Benutzers, wenn die reale Platte (208) dazu ausgelegt ist, den Fuß des Benutzers zu halten, und sich jeder der zweiten Erfassungsaktuatoren an einem anderen Finger oder Zeh der Proxy-Hand oder des Proxy-Fußes befindet.

3. System (200) nach Anspruch 2, wobei die reale Platte (208) und die Proxy-Platte (210) jeweils eine Vielzahl von Fingerclips (504) umfassen, die an der jeweiligen Hand- oder Fußplatte (208, 210) befestigt sind, wobei jeder der Vielzahl von Fingerclips (504) einen anderen der ersten und der zweiten Erfassungsaktuatoren enthält und dazu ausgelegt ist, einen Finger der Hand des Benutzers zuhalten, wenn die reale Platte (208) dazu ausgelegt ist, die Hand des Benutzers zu halten, oder dazu ausgelegt ist, einen Zeh des Fußes des Benutzers zu halten, wenn die reale Platte (208) dazu ausgelegt ist, den Fuß des Benutzers oder die Proxy-Hand oder den Proxy-Fuß zu halten.

4. System nach Anspruch 2, wobei jeder der ersten und der zweiten Erfassungsaktuatoren ein Zahnstangen- (608) und Ritzel(610)-Aktuator ist, der einen Servomotor (606) umfasst, der dazu ausgelegt ist, das Ritzel (610) zu drehen.

5. System nach Anspruch 1, wobei die Proxy-Hand oder der Proxy-Fuß eine digitale Repräsentation der Hand oder des Fußes des Benutzers auf einem Display ist, wobei das Display auf der Proxy-Platte (210) gehalten wird.

6. System (200), das Folgendes umfasst:
eine Berührungsrückmeldevorrichtung (1004), die eine Vielzahl von ersten Erfassungsaktuatoren umfasst und dazu ausgelegt ist, eine Extremität eines Benutzers zu halten;
eine Proxy-Extremität; und
einen Prozessor, der dazu ausgelegt ist, einen der ersten Erfassungsaktuatoren an einer Stelle der Extremität des Benutzers zu betätigen, die einer identifizierten Stelle der Proxy-Extremität entspricht,
wobei sich die Extremität des Benutzers und die Proxy-Extremität in visuell getrennten Sichtfeldern befinden, und
wobei die Vielzahl von ersten Erfassungsaktuatoren dazu ausgelegt sind, eine Erfassungsreaktion des Benutzers zu bewirken, wenn sie betätigt werden.

7. System (200) nach Anspruch 6, das ferner Folgendes umfasst:
einen Lesestift (1002),
wobei der Prozessor dazu ausgelegt ist, die identifizierte Stelle der Proxy-Extremität auf Basis einer Nähe zur Proxy-Extremität oder einer Berührung des Lesestifts (1002) derselben zu bestimmen.

8. System (200) nach Anspruch 6, wobei der Prozessor ferner zu Folgendem ausgelegt ist:
Vorhersagen der identifizierten Stelle der Proxy-Extremität auf Basis einer Bewegung des Lesestifts (1002) oder der Nähe des Lesestifts (1002) zur Proxy-Extremität; und
Betätigen des einen der ersten Erfassungsaktuatoren, bevor der Lesestift (1002) die Proxy-Extremität berührt.

9. System (200) nach Anspruch 6, wobei die Proxy-Extremität eine digitale Repräsentation der Extremität des Benutzers ist.

10. System nach Anspruch 6, wobei die Berührungsrückmeldevorrichtung (1004) dazu ausgelegt ist, eine Hand oder einen Fuß des Benutzers zu halten.

## Revendications

1. Système (200) comprenant :
un cadre inférieur (202) ;
un cadre supérieur (204) fixé de manière rotative au cadre inférieur (202) ;
un plaque réelle (208) fixée au cadre inférieur (202) et configurée pour maintenir une main ou un pied d'un utilisateur ;
une plaque mandataire (210) fixée au cadre supérieur (204) ; et
une main mandataire maintenue sur la plaque mandataire (210) lorsque la plaque réelle (208) est configurée pour maintenir la main de l'utilisateur, ou un pied mandataire maintenu sur la plaque mandataire (210) lorsque la plaque réelle (208) est configurée pour maintenir le pied de l'utilisateur,
dans lequel la plaque réelle (208) comprend une pluralité de premiers actionneurs sensoriels, et la plaque mandataire (210) comprend une pluralité de deuxièmes actionneurs sensoriels, et
dans lequel chacun des deuxièmes actionneurs sensoriels se trouve à un emplacement de la main ou du pied mandataire correspondant à un emplacement de l'un des premiers actionneurs sensoriels de la main ou du pied de l'utilisateur ; et
un processeur configuré pour :
faire fonctionner simultanément l'un des premiers actionneurs sensoriels et l'un des deuxièmes actionneurs sensoriels à l'emplacement correspondant de la main ou du pied mandataire,
faire fonctionner l'un des premiers actionneurs sensoriels et l'un des deuxièmes actionneurs sensoriels à l'emplacement correspondant de la main ou du pied mandataire à différents moments, ou faire fonctionner simultanément l'un des premiers actionneurs sensoriels à un emplacement de la main ou du pied de l'utilisateur et l'un des deuxièmes actionneurs sensoriels à un emplacement différent de la main ou du pied mandataire,
dans lequel un angle entre le cadre inférieur (202) et le cadre supérieur (204) est supérieur à zéro degré, de sorte que lorsque les actionneurs sensoriels sont actionnés, la main ou le pied de l'utilisateur et les premiers actionneurs sensoriels ne soient pas visibles pour l'utilisateur et que la main ou le pied mandataire et les deuxièmes actionneurs sensoriels soient visibles pour l'utilisateur.

2. Système (200) selon la revendication 1, dans lequel chacun des premiers actionneurs sensoriels se trouve à un doigt différent de la main de l'utilisateur lorsque la plaque réelle (208) est configurée pour maintenir la main de l'utilisateur ou se trouve à un orteil différent du pied de l'utilisateur lorsque la plaque réelle (208) est configurée pour maintenir le pied de l'utilisateur, et chacun des deuxièmes actionneurs sensoriels se trouve à un doigt ou à un orteil de la main ou du pied mandataire.

3. Système (200) selon la revendication 2, dans lequel la plaque réelle (208) et la plaque mandataire (210) comprennent chacune une pluralité de pinces de doigts (504) fixées à la plaque de main ou de pied (208, 210) respective, chacune de la pluralité de pinces de doigts (504) logeant un actionneur sensoriel différent des premiers et deuxièmes actionneurs sensoriels et étant configurée pour maintenir un doigt de la main de l'utilisateur lorsque la plaque réelle (208) est configurée pour maintenir la main de l'utilisateur, ou étant configurée pour maintenir un orteil du pied de l'utilisateur lorsque la plaque réelle (208) est configurée pour maintenir le pied de l'utilisateur ou la main ou le pied mandataire.

4. Système selon la revendication 2, dans lequel chacun des premiers et deuxièmes actionneurs sensoriels est un actionneur à crémaillère (608) et pignon (610) comprenant un servomoteur (606) configuré pour faire tourner le pignon (610).

5. Système selon la revendication 1, dans lequel la main ou le pied mandataire est une représentation numérique de la main ou du pied de l'utilisateur sur un affichage, l'affichage étant maintenu sur la plaque mandataire (210).

6. Système (200) comprenant :
un dispositif de retour tactile (1004) comprenant une pluralité de premiers actionneurs sensoriels et configuré pour maintenir une extrémité d'un utilisateur ;
une extrémité mandataire ; et
un processeur configuré pour faire fonctionner l'un des premiers actionneurs sensoriels à un emplacement de l'extrémité de l'utilisateur correspondant à un emplacement identifié de l'extrémité mandataire,
dans lequel l'extrémité de l'utilisateur et l'extrémité mandataire se trouvent dans des champs de vision visuellement séparés, et
dans lequel la pluralité de premiers actionneurs sensoriels sont configurés pour induire une réponse sensorielle de l'utilisateur lorsqu'ils sont actionnés.

7. Système (200) selon la revendication 6, comprenant en outre :
une baguette (1002),
dans lequel le processeur est configuré pour déterminer l'emplacement identifié de l'extrémité mandataire sur la base d'une proximité de la baguette (1002) de l'extrémité mandataire ou d'un contact de la baguette (1002) sur l'extrémité mandataire.

8. Système (200) selon la revendication 6, dans lequel le processeur est en outre configuré pour :
prédire l'emplacement identifié de l'extrémité mandataire sur la base d'un déplacement de la baguette (1002) sur l'extrémité mandataire ou de la proximité de la baguette (1002) de l'extrémité mandataire ; et
faire fonctionner l'un des premiers actionneurs sensoriels avant que la baguette (1002) ne touche l'extrémité mandataire.

9. Système (200) selon la revendication 6, dans lequel l'extrémité mandataire est une représentation numérique de l'extrémité de l'utilisateur.

10. Système selon la revendication 6, dans lequel le dispositif de retour tactile (1004) est configuré pour maintenir une main ou un pied de l'utilisateur.
